# EUROPEAN PATENT APPLICATION

(11) **EP 3 477 658 A1**
(43) Date of publication of application: **01.05.2019**
(21) Application number: 17198799.3
(22) Date of filing: 27.10.2017
(51) Int. Cl.: G16H 50/70

(54) **A METHOD AND APPARATUS FOR OPTIMISING INTERVENTION**

(71) Applicant: Koninklijke Philips N.V., 5656 AE Eindhoven (NL)
(72) Inventor: JIANG, Jie, 5656 AE Eindhoven (NL); HUO, Yong, 5656 AE Eindhoven (NL); CHAN, Tak Ming, 5656 AE Eindhoven (NL); ZHU, Qin, 5656 AE Eindhoven (NL); YIN, Bin, 5656 AE Eindhoven (NL)
(74) Representative: de Haan, Poul Erik

(57) **Abstract**

There is provided a computer-implemented method for generating an optimised intervention interval for a subject. Data associated with the subject is acquired (202). A predicted intervention interval of the subject is estimated (204) based on the data and an intervention regression model. For each predetermined intervention interval, a clinical event coverage value and/or an interval error value is estimated (206) based on the data and a risk prediction model. An intervention interval is indicative of a frequency at which an intervention is provided to the subject. A clinical event coverage value is indicative of a likelihood of preventing /alleviating a future clinical event. An interval error value is indicative of a delay between the predicted and the respective predetermined intervention interval. An optimisation criterion associated with the clinical event coverage value and/or the interval error value is acquired. A intervention interval for which the optimisation criterion is satisfied is selected.

## Description

### TECHNICAL FIELD OF THE INVENTION

The invention relates to the field of subject intervention optimisation and, in particular, to a method and apparatus for generating an optimised intervention interval for a subject.

### BACKGROUND TO THE INVENTION

In post-hospital (post-discharge) management systems currently available, a group of patients is managed by a tele-nurse, health manager, general practitioner (GP), or other primary care provider, who has the responsibility to monitor patient data and provide interventions for patients with lower care plan execution compliance. In some of these post-hospital solutions, a monitoring system may be provided so as to allow an overview of a patient list with the priorities of compliance. In order to optimise the use of limited resources in a clinical environment, it is preferable if primary care providers pay more attention to patients who require intervention, such as by providing an out-patient visit. Currently, monitoring systems only provide basic prioritising functionalities, which are based on a total compliance score of a subject to a care plan (for example, a weighted sum of the compliance score in different categories of the subject, such as whether the patient is taking medication regularly), and this total compliance score does not fully take into account the necessary levels of interventions for different clinical events (e.g. blood pressure too high or shortness of breath).

To explain in further detail, in some of the currently available post-hospital solutions, the patient list is prioritised or optimised based on one category of clinical stratification, in which users can indicate their preferred stratification category. However, these solutions cannot provide an overall priority based on multiple categories and, in some cases, clinical events which require minimal interventions are neglected. In some other solutions, the priorities are set by the total number of clinical events. However, the total number of clinical events are sometimes not indicative of the necessity of intervention. In some other solutions, the priorities are set based on the respective predefined weights assigned to different clinical events. However, these solutions do not consider changes in patient compliance caused by interventions.

### SUMMARY OF THE INVENTION

As noted above, the prioritising functionalities in current monitoring systems for post-hospital management or chronic disease management do not fully take into account the necessary levels of interventions for different clinical events. One of the key reasons for this is that the necessary level of interventions in a care plan is not only based on the total compliance score of a subject but also depends on the effects of the interventions. For example, it is not cost-effective to monitor patients by making phone calls or requesting an out-patient visit, if such efforts are not effective in improving compliance. Nevertheless, it is necessary to provide intervention to a patient when clinical events are prone to happen, even if the patient is not compliant. Moreover, it is also important to provide intervention at optimum intervals.

It would therefore be advantageous to provide a method for generating an optimised list for patient intervention, which also takes into account the quantitative effects of clinical events, patient intervention, and optimum intervals for providing interventions. It would also be advantageous to model the trade-offs between regression of intervention interval and risk prediction of compiled clinical events to provide a quantitative optimised guide for providing necessity level of interventions. To better address one or more of these concerns, in a first aspect, a method for generating an optimised intervention interval for a subject is provided. The method comprises acquiring data associated with the subject, estimating a predicted intervention interval of the subject based on the acquired data and an intervention regression model, and estimating, for each of a plurality of predetermined intervention intervals, at least one of a clinical event coverage value and an interval error value based on the acquired data and a risk prediction model. An intervention interval is indicative of a frequency at which an intervention is provided to the subject, a clinical event coverage value is indicative of a likelihood of preventing or alleviating a future clinical event, and an interval error value is indicative of a delay between the predicted intervention interval of the subject and the respective predetermined intervention interval. The method further comprises acquiring an optimisation criterion associated with one or both of the clinical event coverage value and the interval error value and selecting one of the predetermined intervention intervals for which the optimisation criterion is satisfied.

In some embodiments, the optimisation criterion may be a threshold value associated with one or both of the clinical event coverage value and the interval error value.

In some embodiments, the optimisation criterion is input by a user or predetermined based on at least one of: resources available at a clinical environment, a number of subjects in a clinical environment, a number of caregivers in a clinical environment, a capacity of a clinical environment, and a cost of labour.

In some embodiments, the acquired data associated with the subject may relate to one or both of an attribute present in the intervention regression model and the risk prediction model.

In some embodiments, the acquired data associated with the subject may comprise at least one previous clinical event of the subject, and the predicted intervention interval may be estimated based on historical data of at least one other subject who is associated with the same or similar previous clinical event in the intervention regression model.

In some embodiments, one or both of the intervention regression model and the risk prediction model may be constructed from historical medical data relating to a plurality of subjects. In these embodiments, the historical medical data may comprise at least one attribute of the plurality of subjects and at least one of: a previous clinical event, a compliance to a care plan, a diagnosis, a previous intervention, and a prescribed medication.

In some embodiments, the method may further comprise collecting data relating to at least one of: a subsequent clinical event of the subject, rehabilitation of the subject, and a compliance of the subject to a care plan, and training at least one of the intervention regression model and the risk prediction model using the collected data.

In some embodiments, the method may further comprise estimating a clinical event risk value for the subject based on the acquired data and the risk prediction model, wherein the clinical event coverage value is estimated based on the clinical event risk value for the subject and the respective predetermined intervention interval.

In some embodiments, the method may further comprise controlling a user interface to display the selected predetermined intervention interval for which the optimisation criterion is satisfied. In some of these embodiments, the selected predetermined intervention interval may be displayed in a highlighted format among the plurality of predetermined intervention intervals.

In some embodiments, the method may further comprise acquiring information related to one or both of an amount of resources and effort required to provide intervention at the selected predetermined intervention interval for which the optimisation criterion is satisfied, and controlling a user interface to display the acquired information related to one or both of the amount of resources and effort required. In some of these embodiments, one or both of the amount of resources and effort required to provide intervention may be based on a user-defined cost of healthcare activities.

In some embodiments, the interval error value for a predetermined intervention interval may be estimated by: estimating a mean absolute error of the predicted intervention interval based on the intervention regression model; comparing the predetermined intervention interval with the predicted intervention interval; estimating the interval error value by increasing the mean absolute error if the predetermined intervention interval is larger than the predicted intervention interval; and estimating the interval error value by decreasing the mean absolute error if the predetermined intervention interval is smaller than the predicted intervention interval.
According to a second aspect, there is provided a computer program product comprising a computer readable medium, the computer readable medium having computer readable code embodied therein, the computer readable code being configured such that, on execution by a suitable computer or processor, the computer or processor is caused to perform the method described above.

According to a third aspect, there is provided an apparatus for generating an optimised intervention interval for a subject, the apparatus comprising a processor configured to: acquire data associated with the subject; estimate a predicted intervention interval of the subject based on the acquired data and an intervention regression model; estimate, for each of a plurality of predetermined intervention intervals, at least one of a clinical event coverage value and an interval error value based on the acquired data and a risk prediction model, wherein an intervention interval is indicative of a frequency at which an intervention is provided to the subject, a clinical event coverage value is indicative of a likelihood of preventing or alleviating a future clinical event, and an interval error value is indicative of a delay between the predicted intervention interval of the subject and the predetermined intervention interval; acquire an optimisation criterion associated with one or both of the clinical event coverage value and the interval error value; and select one of the predetermined intervention intervals for which the optimisation criterion is satisfied.

According to the aspects and embodiments described above, the limitations of existing techniques are addressed. There is thus provided an improved method and apparatus for generating an optimised intervention interval for a subject.

These and other aspects will be apparent from and elucidated with reference to the embodiment(s) described hereinafter.

### BRIEF DESCRIPTION OF THE DRAWINGS

For a better understanding of the embodiments, and to show more clearly how they may be carried into effect, reference will now be made, by way of example only, to the accompanying drawings, in which:
Figure 1 is a block diagram of an apparatus for generating an optimised intervention interval for a subject according to an embodiment;
Figure 2 illustrates a method for generating an optimised intervention interval for a subject according to an embodiment; and
Figure 3 is a schematic diagram illustrating data flow in a method according an example embodiment.

### DETAILED DESCRIPTION OF THE EMBODIMENTS

As noted above, there is provided an improved method and apparatus for generating an optimised intervention interval for a subject, which overcomes existing problems.

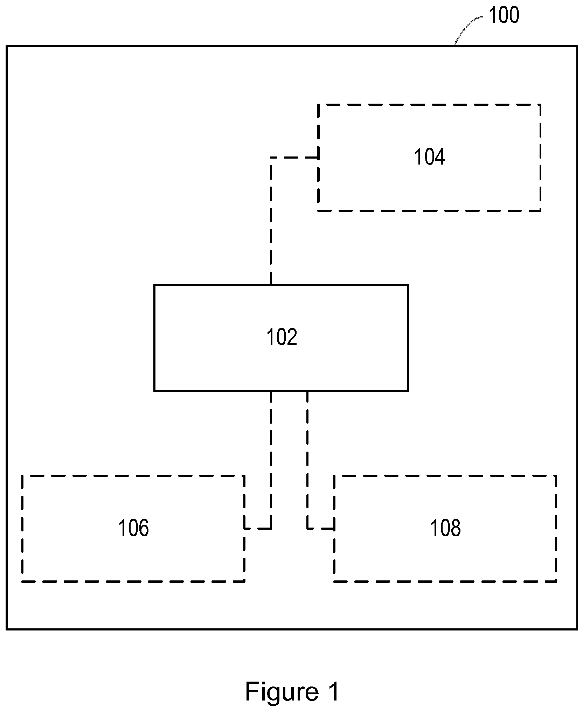

**Figure 1** shows a block diagram of an apparatus 100 according to an embodiment that can be used for generating an optimised intervention interval for a subject. An intervention may be an effort that promotes behaviour of the subject to improve their mental and/or physical health, or that discourages or reframes behaviour(s) with health risks.

As illustrated in Figure 1, the apparatus 100 comprises a processor 102 that controls the operation of the apparatus 100 and that can implement the method described herein. The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein.

Briefly, the processor 102 is configured to acquire data associated with the subject and estimate a predicted intervention interval of the subject based on the acquired data and an intervention regression model. The processor 102 is also configured to estimate, for each of a plurality of predetermined intervention intervals, at least one of a clinical event coverage value and an interval error value based on the acquired data and a risk prediction model. An intervention interval is indicative of a frequency at which an intervention is provided to the subject. A clinical event coverage value is indicative of a likelihood of preventing or alleviating a future clinical event. An interval error value is indicative of a delay between the predicted intervention interval of the subject and the respective predetermined intervention interval. The processor 102 is further configured to acquire an optimisation criterion associated with one or both of the clinical event coverage value and the interval error value and to select one of the predetermined intervention intervals for which the optimisation criterion is satisfied.

In some embodiments, the apparatus 100 may further comprise at least one user interface 104. Alternatively and in addition, at least one user interface 104 may be external to (i.e. separate to or remote from) the apparatus 100. For example, at least one user interface 104 may be part of another device. A user interface 104 may be for use in providing a user of the apparatus 100 with information resulting from the method described herein. For example, the processor 102 may be configured to control one or more user interfaces 104 to render (or output or display) the selected predetermined intervention interval for which the optimisation criterion is satisfied. Alternatively or in addition, a user interface 104 may be configured to receive a user input. In other words, a user interface 104 may allow a user of the apparatus 100 to manually enter instructions, data, or information. The processor 102 may be configured to acquire the user input from one or more user interfaces 104.

A user interface 104 may be any user interface that enables rendering (or output or display) of information to a user of the apparatus 100. Alternatively or in addition, a user interface 104 may be any user interface that enables a user of the apparatus 100 to provide a user input, interact with and/or control the apparatus 100. For example, the user interface 104 may comprise one or more switches, one or more buttons, a keypad, a keyboard, a touch screen or an application (for example, on a tablet or smartphone), a display screen, a graphical user interface (GUI) or other visual rendering component, one or more speakers, one or more microphones or any other audio component, one or more lights, a component for providing tactile feedback (e.g. a vibration function), or any other user interface, or combination of user interfaces.

In some embodiments, the apparatus 100 may comprise a memory 106 configured to store program code that can be executed by the processor 102 to perform the method described herein. Alternatively or in addition, one or more memories 106 may be external to (i.e. separate to or remote from) the apparatus 100. For example, one or more memories 106 may be part of another device. A memory 106 can be used to store information, data, signals and measurements acquired or made by the processor 102 of the apparatus 100. For example, a memory 106 may be used to store (for example, in a local file) data associated with a subject or a plurality of subjects. The processor 102 may be configured to control a memory 106 to store the data associated with the subject or the plurality of subjects.

In some embodiments, the apparatus 100 may comprise a communications interface (or circuitry) 108 for enabling the apparatus 100 to communicate with any interfaces, memories and/or devices that are internal or external to the apparatus 100. The communications interface 108 may communicate with any interfaces, memories and/or devices wirelessly or via a wired connection. For example, the communications interface 108 may communicate with the one or more user interfaces 104 wirelessly or via a wired connection. Similarly, the communications interface 108 may communicate with the one or more memories 106 wirelessly or via a wired connection.

It will be appreciated that Figure 1 only shows the components required to illustrate an aspect of the apparatus and, in a practical implementation, the apparatus 100 may comprise alternative or additional components to those shown. For example, in some embodiments, the apparatus 100 may comprise a battery or other power supply for powering the apparatus 100 or means for connecting the apparatus 100 to a mains power supply.

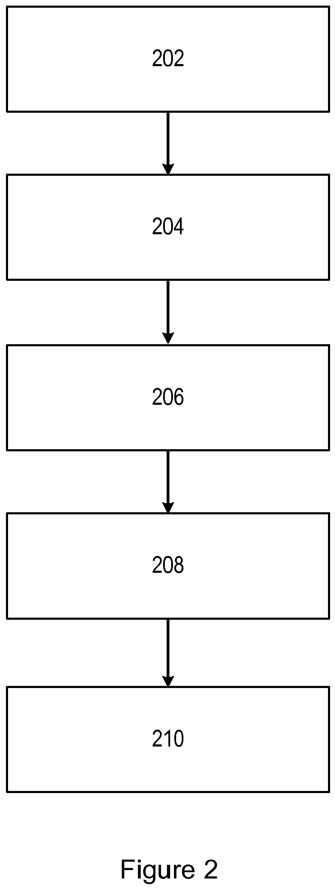

**Figure 2** illustrates a computer-implemented method 200 for generating an optimised intervention interval for a subject according to an embodiment. The illustrated method 200 can generally be performed by or under the control of the processor 102 of the apparatus 100.

With reference to Figure 2, at block 202, data associated with the subject is acquired. In some embodiments, this data is acquired by the processor 102 of the apparatus 100 from a memory 106, which may be a memory of the apparatus 100 or a memory external to the apparatus 100. The data associated with the subject may relate to an attribute present in one or both of an intervention regression model and a risk prediction model. For example, the data associated with the subject may relate to or comprise any one or more of the following attributes: an age of the subject, a gender of the subject, a height of the subject, a weight of the subject, a smoking status of the subject, a family history of the subject, a medical history of the subject, or any other attribute of the subject, or any combination of attributes of the subject. In some embodiments, the acquired data may comprise at least one previous clinical event of the subject.

A clinical event may be any event (for example, any incident or situation) where the health of the subject is adversely affected (for example, adversely impacted or influenced). Examples of a clinical event include, but are not limited to, an increase in the severity, acuity and/or stage of a disease in the subject, an onset or worsening of a medical condition for the subject, a detrimental impact on the well-being of the subject, a deterioration of a health condition of the subject (for example, a deterioration in one or more physiological characteristics or vital signs of the subject), a death of the subject, or any other event where the health of the subject is adversely affected, or any combination thereof. In some embodiments, a clinical event may be any event that necessitates or requires intervention from a medical practitioner or another person.

As will be explained in further detail below, one or both of the intervention regression model and the risk prediction model will be used at block 204 and block 206 of Figure 2. One or both of the intervention regression model and the risk prediction model may be constructed from historical medical data relating to a plurality of subjects (not including the subject for which the optimised intervention interval is generated in this embodiment).

In some embodiments, the historical medical data relating to the plurality of subjects may comprise at least one attribute (e.g. age, gender, height, weight, etc.) of the plurality of subjects and also at least one of: a previous clinical event, a compliance to a care plan, a diagnosis, a previous intervention, and a prescribed medication. A previous clinical event may be characterised by a clinical event level or a clinical event threshold, e.g. a "high blood pressure" level if in the last 5 days, the value of systolic blood pressure of the subject is between 140 and 160 more than 3 times. A compliance to a care plan may be characterised by a compliance score, e.g. 80% compliance score to a care plan. A diagnosis may be characterised by a statement indicative of a disease of the subject, e.g. coronary artery disease. A previous intervention may be characterised by a statement indicative of an action performed to provide a healthcare intervention to the subject, e.g. percutaneous coronary intervention. A care plan may contain instructions to a subject regarding their health condition, for example, a timetable for doing physical exercise or instructions for taking medication.

In some embodiments where the historical medical data for constructing one or both of the intervention regression model and the risk prediction model comprises a respective previous clinical event of the plurality of subjects, the previous clinical event(s) may be represented by clinical event label(s) each corresponding to a clinical event, and each of the plurality of subjects may be associated with at least one of the plurality of clinical event labels. By using this historical medical data relating to the plurality of subjects, one or both of the intervention regression model and the risk prediction model may provide a prediction relating to the optimised intervention interval of the subject. This will be explained in further detail below.

At block 204 of Figure 2, a predicted intervention interval of the subject is estimated based on the data acquired at block 202 of Figure 2 and an intervention regression model. A predicted intervention interval in the current context refers to an intervention interval predicted only using the intervention regression model and therefore has not been fully optimised by taking into consideration predicted risk(s) of future clinical events.

In some embodiments, the intervention regression model may perform prediction analysis using regression data mining technique(s). For example, this may provide an estimation of a predicted intervention interval for a subject on the basis of the similarity between the clinical event(s) that occurred to the subject (or that the subject experienced) and the clinical event(s) that occurred to at least one of the plurality of subjects (or that the at least one of the plurality of subjects experienced) whose historical medical data is used for constructing the intervention regression model.

As mentioned above, in some embodiments, the data acquired at block 202 of Figure 2 may comprise at least one previous clinical event of the subject, e.g. an onset of a medical condition of the subject. In these embodiments, at block 204 of Figure 2, the processor 102 may be configured to estimate the predicted intervention interval, using the intervention regression model, based on historical data of at least one other subject (among the plurality of subjects whose historical medial data is used to construct the intervention regression model) who is associated with the same or similar previous clinical event, i.e. an onset of the same/similar medical condition.

At block 206 of Figure 2, for each of a plurality of predetermined intervention intervals, at least one of a clinical event coverage value and an interval error value is estimated, based on the data acquired at block 202 of Figure 2 and the risk prediction model. The risk prediction model is a classification model which allows a clinical event coverage value to be estimated on the basis of historical data relating to a plurality of subjects (not including the current subject for which the intervention interval is optimised). As mentioned above, the clinical event coverage value is indicative of a likelihood of preventing or alleviating a future clinical event, and an interval error value is indicative of a delay between the predicted intervention interval of the subject and the respective predetermined intervention interval. In some embodiments, the plurality of predetermined intervention intervals may be based on data associated with the subject that is acquired at block 202 of Figure 2. For example, in some embodiments, the acquired data associated with the subject may comprise information related to an availability of the subject (e.g. whether the subject is available to receive an intervention on specific days), and the plurality of predetermined intervention intervals may be based on this information related to the availability of the subject.

In some embodiments, the interval error value for a predetermined intervention interval may be estimated by comparing the predetermined intervention interval with the predicted intervention interval. In more detail, in these embodiments, a mean absolute error of the predicted intervention interval may be estimated based on the intervention regression model. If the predetermined intervention interval is larger than the predicted intervention interval, the interval error value may be estimated by increasing the mean absolute error; and if the predetermined intervention interval is smaller than the predicted intervention interval, the interval error value may be estimated by decreasing the mean absolute error. The degree of increasing or decreasing the mean absolute error may be dependent on a degree of difference between the predetermined intervention interval and the predicted intervention interval.

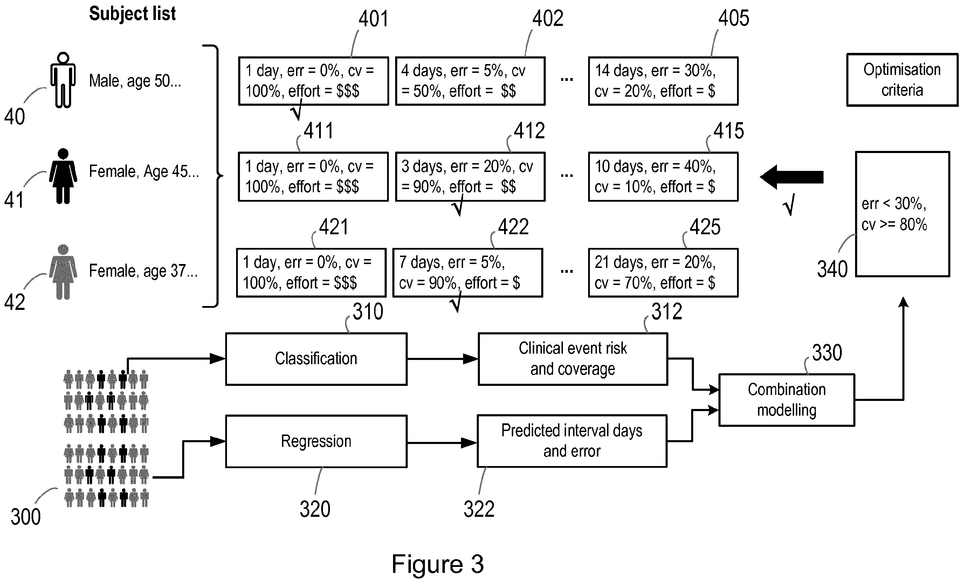

In some embodiments, one or both of the estimated clinical event coverage value (cv) and the estimated interval error value (err) may be expressed as a percentage. For example, an estimated clinical event coverage value for a certain subject may be expressed as "cv = 60%" and an estimated interval error value may be expressed as "err = 20%". This is shown in the exemplary data flow diagram in Figure 3, which will be explained in more detail later.

Although not illustrated in Figure 2, in some embodiments, prior to block 206 of Figure 2, a clinical event risk value for the subject may be estimated based on the acquired data at block 202 and the risk prediction model. In these embodiments, at block 206 of Figure 2, the clinical event coverage value may be estimated based on the clinical event risk value for the subject and a predetermined risk threshold, in addition to the use of the risk prediction model. Specifically, in these embodiments, the clinical event coverage value may be estimated by considering a difference between a respective predetermined intervention interval and the predicted intervention interval (estimated at block 204 of Figure 2). To explain in more detail, if a respective predetermined intervention interval is smaller than the predicted intervention interval, the clinical event coverage value may be higher. In contrast, if a respective predetermined intervention interval is larger than the predicted intervention interval, the clinical event coverage value may be lower. The estimation of the clinical event coverage value in these embodiments may be based on one or both of a weight multiplication and historical intervention intervals.

For example, in some embodiments, a clinical event risk value for a subject may be represented by a risk score between the value of 0 and 1. Based on the risk prediction model and the acquired data associated with the subject, the subject may be assigned an exemplary risk score of 0.2 (clinical event risk value) by interpolating from data of other subject(s) with similar age, and/or similar medication, and/or similar previous clinical event, etc. As an example, in some embodiments, an exemplary risk score of 0.2 may indicate that 20% (or around 20%) of the plurality of subjects whose historical medical data is used to construct the intervention regression model and/or the risk prediction model and who have similar attribute(s) to those of the subject develop a subsequent clinical event (e.g. a serious adverse clinical event). This risk score of 0.2 can then be converted into a clinical event coverage value by considering, using the risk prediction model, how likely it is a future clinical event can be prevented or alleviated if intervention is provided at the respective predetermined intervention interval. For example, for an exemplary subject with a risk score of 0.2, their clinical event coverage value may be 100% for a predetermined intervention interval of 1 day (meaning that the likelihood of preventing or alleviating the subsequent clinical event is 100%), and their clinical event coverage value may be 90% for a predetermined intervention interval of 3 days (meaning that the likelihood of preventing or alleviating the subsequent clinical event is 90%). In other words, the clinical event coverage value decreases when the predetermined intervention interval increases, since a less frequent intervention would typically lead to a higher likelihood of an occurrence of a future clinical event.

At block 208 of Figure 2, an optimisation criterion associated with one or both of the clinical event coverage and the interval error value is acquired. This optimisation criterion may be a threshold value associated with one or both of the clinical event coverage value and the interval error value. For example, in some embodiments, the optimisation criterion may be "clinical event coverage value bigger than or equal to 85%", or "interval error value smaller than 20%".

In some embodiments, the optimisation criterion may be input by a user (e.g. via a user interface 104, which may be a user interface of the apparatus 100 or a user interface external to the apparatus 100) or predetermined based on at least one of: resources available at a clinical environment, a number of subjects in a clinical environment, a number of care givers in a clinical environment, a capacity of a clinical environment, and a cost of labour. The optimisation criterion may be set so as to minimise the resources and/or efforts required to provide intervention to the subject, while maximising the clinical event coverage value, i.e. the likelihood of preventing or alleviating a future clinical event.

At block 210 of Figure 2, one of the predetermined intervention intervals for which the optimisation criterion is satisfied is selected. This selected predetermined intervention interval may be referred as the "optimised intervention interval" for the subject, since this intervention interval is generated on the basis of a combination and a trade-off of two different techniques, i.e. the regression data-mining technique used at the intervention regression model and the classification data-mining technique used at the risk prediction model.

In some embodiments, the selected predetermined intervention interval for the subject for which the optimisation criterion is satisfied may be displayed via control of a user interface 104 by the processor 102. As mentioned earlier, the user interface 104 may be a user interface of the apparatus 100 or an external user interface. Also, in some of these embodiments, the selected predetermined intervention for the subject for which the optimisation criterion is satisfied may be displayed in a highlighted format among the plurality of predetermined intervention intervals.

In some embodiments, the method may further comprise acquiring information related to one or both of an amount of resources and effort required to provide intervention at the selected predetermined intervention interval for which the optimisation criterion is satisfied. In these embodiments, the method may further comprise controlling a user interface 104 (which may be a user interface of the apparatus 100 or a separate user interface) to display the acquired information. One or both of the amount of resources and effort required to provide the intervention may be based on a user-defined cost of healthcare activities. As an example, one or both of the amount of resources and effort required may depend on a type of intervention provided, for example, arranging a visit to the subject by a care giver (e.g. a nurse) may require more resources and/or effort than providing a reminder (e.g. a phone call) to the subject to comply to the care plan. As the amount of resources and/or effort required also depends on a number of other factors, such as cost of labour, this may be defined by a user prior to performing the method of the present embodiment.

In some embodiments, the method described herein may be applied to a plurality of subjects simultaneously or sequentially. In some of these embodiments, the selected predetermined intervention interval for each of the plurality of subjects may be displayed together at a user interface 104, which may be the user interface 104 of the apparatus 100 or a user interface external to the apparatus 100. Therefore, an overview of different generated optimised intervention intervals for each of the plurality for subjects can be provided to a user (e.g. a physician).

Although not illustrated in Figure 2, subsequent to block 210 of Figure 2, the method may further comprise collecting data relating to the subject for which the optimised intervention interval is generated. For example, the collected data may relate to at least one of: a subsequent clinical event of the subject, rehabilitation of the subject, and a compliance of the subject to a care plan. The collected data of the subject can be used to train one or both of the intervention regression model and the risk prediction model. For example, the collected data can be used as part of the historical medical data for constructing one or both of the intervention regression model and the risk prediction model, so as to update and/or improve the accuracy of at least one of these models by increasing sample size. Moreover, in these embodiments, the collected data together with the data acquired at block 202 of Figure 2 may be used for training one or both of the intervention regression model and the risk prediction model.

**Figure 3** is a schematic diagram illustrating data flow in a method according an example embodiment.

As shown in Figure 3, there is provided a sample 300 comprising a plurality of subjects. The sample 300 represents data associated with the plurality of subjects. More specifically, in this example embodiment, the sample 300 represents historical medical data associated with the plurality of subjects. The data associated with the plurality of subjects is used to construct an intervention regression model and a risk prediction model. As mentioned earlier, the data associated with the plurality of subjects may, for example, comprise at least one attribute of the plurality of subjects (e.g. age, gender, height, weight, etc.) and at least one of: a previous clinical event, a compliance to a care plan, a diagnosis, a previous intervention, and a prescribed medication. As an example, in some embodiments, the historical medical data associated with the plurality of subjects may comprise an age and a previous clinical event. For example, the historical medical data may include, for each of the plurality of subjects, an age of the subject and a previous clinical event that occurred to the subject (e.g. 50 years old, heart attack; 42 years old, gastrointestinal bleeding; etc.).

The historical medical data is used to construct the risk prediction model and intervention regression model in this example embodiment. As shown in Figure 3, this is represented by block 310 ("classification") and block 320 ("regression"), where classification data-mining technique(s) and regression data-mining technique(s) are applied to construct the risk prediction model and the intervention regression model. The risk prediction model and the intervention regression model are respectively represented in Figure 3 by block 312 ("clinical event risk and coverage") and block 322 ("predicted interval days and error"). Block 330 ("combination modelling") in Figure 3 represents the method in which both models are employed so as to generate an optimised intervention interval for a subject or for multiple subjects.

In the example embodiment illustrated in Figure 3, there is provided a subject list which contains examples of subjects 40, 41, 42 for which an optimised intervention interval is to be generated respectively. The subject list contains a first subject 40 who is a 50 year old male, a second subject 41 who is a 45 year old female, and a third subject 42 who is a 37 year old female.

According to the method illustrated in Figure 2, for each of the first subject 40, the second subject 41, and the third subject 42, a predicted intervention interval of the subject is estimated (block 204 of Figure 2). As described earlier with reference to Figure 2, the predicted intervention interval of the subject is estimated based on acquired data associated with the subject (which is acquired at block 202 of Figure 2) and the intervention regression model. In this example embodiment, for each of these subjects 40, 41, 42, a clinical event coverage value and an interval error value are estimated for each of a plurality of predetermined intervention intervals (block 206 of Figure 2). As described earlier with reference to Figure 2, the clinical event coverage value and the interval error value are estimated based on the acquired data associated with the subject and the risk prediction model. For example, for the first subject 40 who is a 50 year old male, his predicted intervention interval, clinical event coverage value, and interval error value may be estimated by specifically using historical medical data of male subjects who are of similar age.

In the example embodiment illustrated in Figure 3, the plurality of predetermined intervention intervals are different for different subjects. As shown at blocks 401, 402...405, for the first subject 40, the plurality of predetermined intervention intervals are 1 day, 4 days,...,14 days. As shown at blocks 411, 412...415, for the second subject 41, the plurality of predetermined intervention intervals are 1 day, 3 days... 10 days. As shown at blocks 412, 422...425, for the third subject 42, the plurality of predetermined intervention intervals are 1 day, 7 days...21 days.

In some embodiments, the plurality of predetermined intervention intervals may be arbitrary. In some other embodiments, the plurality of predetermined intervention intervals may be predetermined by a user. In some other embodiments, the plurality of predetermined intervention intervals may be determined based on the acquired data associated with the subject. For example, the plurality of predetermined intervention intervals may be based on a previous clinical event of the subject. In some embodiments, even for different subjects the plurality of predetermined intervention intervals may be the same. Although in this example the plurality of predetermined intervention intervals are characterised in days, it will be appreciated that, in other embodiments, the plurality of predetermined intervention intervals may be characterised in other units of time, e.g. hours.

The estimated clinical event coverage value and interval error value for each of the plurality of predetermined intervention intervals for the first subject 40 are represented by blocks 401, 402...405 of Figure 3. In block 401, it is shown that the estimated clinical event coverage value and interval error value for a predetermined intervention interval for 1 day for the first subject 40 is respectively 100% and 0%. In block 402, it is shown that the estimated clinical event coverage value and interval error value for a predetermined intervention interval for 4 days for the first subject 40 is respectively 50% and 5%. In block 405, it is shown that the estimated clinical event coverage value and interval error value for a predetermined intervention interval for 14 days for the first subject 40 is respectively 20% and 30%.

Similarly, the estimated clinical event coverage value and interval error value for each of the plurality of predetermined intervention intervals for the second subject 41 and the third subject 42 are represented by blocks 411, 412,...405, and 421, 422,...425. In block 411, it is shown that the estimated clinical event coverage value and interval error value for a predetermined intervention interval for 1 day for the second subject 41 is respectively 100% and 0%. In block 412, it is shown that the estimated clinical event coverage value and interval error value for a predetermined intervention interval for 3 days for the second subject 41 is respectively 90% and 20%. In block 415, it is shown that the estimated clinical event coverage value and interval error value for a predetermined intervention interval for 10 days for the second subject 41 is respectively 10% and 40%. In block 421, it is shown that the estimated clinical event coverage value and interval error value for a predetermined intervention interval for 1 day for the third subject 42 is respectively 100% and 0%. In block 422, it is shown that the estimated clinical event coverage value and interval error value for a predetermined intervention interval for 7 days for the third subject 42 is respectively 90% and 5%. In block 425, it is shown that the estimated clinical event coverage value and interval error value for a predetermined intervention interval for 21 days for the third subject 42 is respectively 70% and 20%.

In this example embodiment shown in Figure 3, information related to an amount of resources and/or effort required to provide intervention at each of the plurality of predetermined intervention intervals is also acquired and presented along with the estimated clinical event coverage value and the estimated interval error value. For example, in the example embodiment shown in Figure 3, an amount of resources and/or effort required to provide intervention may be expressed in the form of three different levels, i.e. '$' indicating a low amount of resources and/or effort, '$$' indicating a medium amount of resources and/or effort, and '$$$' indicating a high amount of resources and/or effort.

Based on a number of factors such as: resources available at a clinical environment, a number of subjects in a clinical environment, a number of care givers in a clinical environment, a capacity of a clinical environment, and a cost of labour, the optimisation criteria for a specific clinical environment, such as a hospital, can be determined (block 208 of Figure 2). In this example, the optimisation criteria are: "clinical event coverage value greater than or equal to 80%" and "interval error value less than 30%" (which is represented by block 340 of Figure 3). Using these optimisation criteria, a predetermined intervention interval for each of the subjects 40, 41, 42 is selected (block 210 of Figure 2). In this example embodiment, the selected intervention interval for each of the subjects 40, 41, 42 is displayed in a highlighted format (a 'tick' icon is displayed adjacent to each of the blocks representing the selected intervention intervals 401, 412, 422). Hence, an overview of the optimised intervention intervals (i.e. the selected intervention intervals 401, 412, 422) for each of the plurality for subjects 40, 41, 42 can be provided and presented to a user (e.g. a physician).

There is thus provided an improved method and apparatus for generating an optimised intervention interval for a subject, which overcomes the existing problems.

There is also provided a computer program product comprising a computer readable medium, the computer readable medium having computer readable code embodied therein, the computer readable code being configured such that, on execution by a suitable computer or processor, the computer or processor is caused to perform the method or methods described herein. Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method for generating an optimised intervention interval for a subject, the method comprising:
acquiring (202) data associated with the subject;
estimating (204) a predicted intervention interval of the subject based on the acquired data and an intervention regression model;
estimating (206), for each of a plurality of predetermined intervention intervals, at least one of a clinical event coverage value and an interval error value based on the acquired data and a risk prediction model, wherein an intervention interval is indicative of a frequency at which an intervention is provided to the subject, a clinical event coverage value is indicative of a likelihood of preventing or alleviating a future clinical event, and an interval error value is indicative of a delay between the predicted intervention interval of the subject and the respective predetermined intervention interval;
acquiring (208) an optimisation criterion associated with one or both of the clinical event coverage value and the interval error value; and
selecting (210) one of the predetermined intervention intervals for which the optimisation criterion is satisfied.

2. A computer-implemented method as claimed in claim 1, wherein the optimisation criterion is a threshold value associated with one or both of the clinical event coverage value and the interval error value.

3. A computer-implemented method as claimed in claim 1 or claim 2, wherein the optimisation criterion is input by a user or predetermined based on at least one of: resources available at a clinical environment, a number of subjects in a clinical environment, a number of caregivers in a clinical environment, a capacity of a clinical environment, and a cost of labour.

4. A computer-implemented method as claimed in any of the preceding claims, wherein the acquired data associated with the subject relates to one or both of an attribute present in the intervention regression model and the risk prediction model.

5. A computer-implemented method as claimed in any of the preceding claims, wherein the acquired data associated with the subject comprises at least one previous clinical event of the subject, and the predicted intervention interval is estimated based on historical data of at least one other subject who is associated with the same or similar previous clinical event in the intervention regression model.

6. A computer-implemented method as claimed in any of the preceding claims, wherein one or both of the intervention regression model and the risk prediction model is constructed from historical medical data relating to a plurality of subjects, and wherein the historical medical data comprises at least one attribute of the plurality of subjects and at least one of: a previous clinical event, a compliance to a care plan, a diagnosis, a previous intervention, and a prescribed medication.

7. A computer-implemented method as claimed in any of the preceding claims, the method further comprising:
collecting data relating to at least one of: a subsequent clinical event of the subject, rehabilitation of the subject, and a compliance of the subject to a care plan; and
training at least one of the intervention regression model and the risk prediction model using the collected data.

8. A computer-implemented method as claimed in any of the preceding claims, the method further comprising:
estimating a clinical event risk value for the subject based on the acquired data and the risk prediction model,
wherein the clinical event coverage value is estimated based on the clinical event risk value for the subject and the respective predetermined intervention interval.

9. A computer-implemented method as claimed in any of the preceding claims, the method further comprising:
controlling a user interface (104) to display the selected predetermined intervention interval for which the optimisation criterion is satisfied.

10. A computer-implemented method as claimed in claim 9, wherein the selected predetermined intervention interval is displayed in a highlighted format among the plurality of predetermined intervention intervals.

11. A computer-implemented method as claimed in any of the preceding claims, the method further comprising:
acquiring information related to one or both of an amount of resources and effort required to provide intervention at the selected predetermined intervention interval for which the optimisation criterion is satisfied; and
controlling a user interface (104) to display the acquired information related to one or both of the amount of resources and effort required.

12. A computer-implemented method as claimed in claim 11, wherein one or both of the amount of resources and effort required to provide intervention is based on a user-defined cost of healthcare activities.

13. A computer-implemented method as claimed in any of the preceding claims, wherein the interval error value for a predetermined intervention interval is estimated by:
estimating a mean absolute error of the predicted intervention interval based on the intervention regression model;
comparing the predetermined intervention interval with the predicted intervention interval;
estimating the interval error value by increasing the mean absolute error if the predetermined intervention interval is larger than the predicted intervention interval; and
estimating the interval error value by decreasing the mean absolute error if the predetermined intervention interval is smaller than the predicted intervention interval.

14. A computer program product comprising a computer readable medium, the computer readable medium having computer readable code embodied therein, the computer readable code being configured such that, on execution by a suitable computer or processor, the computer or processor is caused to perform the method as claimed in any of the preceding claims.

15. An apparatus (100) for generating an optimised intervention interval for a subject, the apparatus (100) comprising a processor (102) configured to:
acquire data associated with the subject;
estimate a predicted intervention interval of the subject based on the acquired data and an intervention regression model;
estimate, for each of a plurality of predetermined intervention intervals, at least one of a clinical event coverage value and an interval error value based on the acquired data and a risk prediction model, wherein an intervention interval is indicative of a frequency at which an intervention is provided to the subject, a clinical event coverage value is indicative of a likelihood of preventing/alleviating a future clinical event, and an interval error value is indicative of a delay between the predicted intervention interval of the subject and the predetermined intervention interval;
acquire an optimisation criterion associated with the clinical event coverage value and/or the interval error value; and
select one of the predetermined intervention intervals for which the optimisation criterion is satisfied.
